# FASCICULE DE BREVET EUROPEEN

(11) **EP 0 501 868 B1**
(45) Date de publication et mention de la délivrance du brevet: **17.07.1996**
(21) Numéro de dépôt: 92400473.2
(22) Date de dépôt: 24.02.1992
(51) Int. Cl.: C07C 53/21, C07C 55/32, C10M 133/06, C10M 133/10

(54) **Sels d'amines grasses et d'acides polyfluorocarboxyliques, et leur utilisation comme additifs pour lubrifiants**
Salze von Fettaminen und Polyfluorocarbonsäuren und ihre Verwendung als Schmiermittelzusätze
Salts of fatty amines and polyfluoro carboxylic acids, and use of them as lubricant additives

(30) Priorité: 27.02.1991 FR 9102345
(43) Date de publication de la demande: 02.09.1992
(73) Titulaire: ELF ATOCHEM S.A., F-92800 Puteaux (FR)
(72) Inventeur: Schoch, Elisabeth, Sonchamp, F-78120 Rambouillet (FR); Collette, Christian, F-75005 Paris (FR)
(74) Mandataire: Leboulenger, Jean

(56) Documents cités:
- FR-A- 2 520 377
- US-A- 2 951 051
- US-A- 4 107 055
- JOURNAL OF ORGANIC CHEMISTRY vol. 27, Décembre 1962, EASTON U.S. pages 4491 - 4498; N.O. BRACE: 'LONG CHAIN ALKANOIC AND ALKENOIC ACIDS WITH PERFLUOROALKYL TERMINAL SEGMENTS?

## Description

La présente invention concerne le domaine des produits fluorés et celui des lubrifiants. Elle a plus particulièrement pour objets de nouveaux sels d'acides polyfluorocarboxyliques, utilisables comme additifs anti-usure pour lubrifiants.

L'application de certains dérivés organo-fluorés comme additifs à des compositions lubrifiantes est connue ; par exemple l'application des sels d'amines aliphatiques et d'acides monocarboxyliques perhalogénés tels que l'acide Cl(CF₂CFCl)₃CF₂COOH et l'acide C₇F₁₅COOH a été décrite dans le brevet US 3 565 926, de même que l'application des dérivés obtenus par réaction d'une amine aromatique et d'un acide monocarboxylique perfluoré (par exemple C₇F₁₅COOH) a été divulguée dans le brevet FR 2 026 493. Cependant ces dérivés carboxyliques présentent l'inconvénient de perdre leur efficacité anti-usure en présence d'additifs classiques tels que les additifs dispersants-détergents, soit par suite d'interactions physico-chimiques qui empêchent leur absorption au niveau des surfaces à lubrifier, soit à cause d'interactions chimiques, en particulier lorsque les additifs dispersants-détergents sont des sels de métaux alcalino-terreux neutres ou surbasés.

Comme additifs anti-usure pour lubrifiants, on a également proposé l'emploi d'amines ou d'amino-alcools à chaîne polyfluorée (brevet FR 2 520 377) et celui de dérivés de ces amino-alcools (brevets FR 2 599 378, 2 616 781 et 2 616 783).

On connait aussi certains acides polyfluorocarboxyliques de formules :

R_{F}(CH₂)₁₀COOH (I)

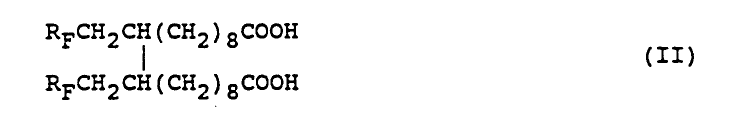

dans lesquelles R_{F} désigne un radical perfluoroalkyle.

L'emploi de ces acides ou de leurs dérivés comme additifs pour lubrifiants ne semble pas avoir encore été envisagé. Selon le brevet US 2 951 051 qui décrit l'acide 11-(perfluorooctyl)-undécanoïque, ses sels de sodium et de potassium et son complexe de chrome (exemples II et V), les acides mono-carboxyliques (I) sont préparés à partir de l'acide 10-undécènoïque et d'un sulfochlorure ou iodure de perfluoroalkyle. Le brevet US 4 107 055, relatif à l'emploi des diacides et de leurs sels alcalins comme agents tensio-actifs fluorés dans des compositions anti-salissures à base de latex de polymères non halogénés pour textiles, indique que les diacides (II) peuvent être préparés par action du zinc sur des iodo-acides R_{F}CH₂CHI(CH₂)ₘCOOH dans l'éthanol.

Il a maintenant été trouvé que les sels formés par une amine grasse avec un mélange des acides polyfluorés (I) et (II) présentent une efficacité anti-usure remarquable, biensupérieure à celle des sels des acides monocarboxyliques perhalogénés et au moins égale, voire supérieure, à celle des additifs du type amine ou amino-alcool.

L'invention a donc comme premier objet les sels formés par une amine grasse avec un mélange comprenant au moins un acide monocarboxylique de formule (I) et au moins un acide dicarboxylique de formule (II), le radical perfluoroalkyle R_{F} pouvant être linéaire ou ramifié et contenir de 4 à 16 atomes de carbone, de préférence 6 à 12.

L'invention a également pour objet l'utilisation de ces sels comme additifs anti-usure pour lubrifiants.

Le mélange d'acides (I) et (II) est avantageusement obtenu en faisant réagir un iodure de perfluoroalkyle R_{F}I sur l'acide 10-undécènoïque en présence de zinc et en milieu dichlorométhane, à une température d'environ 50°C. Après élimination du zinc sous forme de chlorure, le mélange d'acides mono- et dicarboxyliques est extrait à l'éther. Quand on met en oeuvre des quantités sensiblement équimolaires de R_{F}I, d'acide 10-undécènoïque et de zinc, on obtient généralement un mélange dont la teneur en monoacide (I) varie entre environ 60 et 75 % en moles, cette teneur étant d'autant plus grande que le nombre d'atomes de carbone du R_{F}I de départ est petit. Si on le désire, on peut orienter la réaction vers la formation majoritaire d'acide dicarboxylique (II) en utilisant une quantité moindre de zinc, par exemple 0,5 mole de zinc par mole de R_{F}I.

Pour des raisons économiques et pratiques, il peut s'avérer intéressant de partir d'un mélange industriel d'iodures de perfluoroalkyle, ce qui conduit à un mélange complexe de plusieurs monoacides et diacides présentant des radicaux R_{F} différents.

L'amine grasse à utiliser pour former les sels selon l'invention peut être choisie parmi les amines aliphatiques ou cycloaliphatiques primaires, secondaires ou tertiaires, contenant au total de 12 à 24 atomes de carbone, de préférence de 16 à 20 atomes de carbone. Comme exemples non limitatifs de telles amines, on peut citer plus particulièrement la N,N-bis(2-éthylhexyl)amine, la dioctylamine, l'oléylamine et l'octadécylamine. Bien entendu, on ne sortirait pas du cadre de la présente invention en utilisant un mélange d'amines grasses.

Les sels selon l'invention peuvent être préparés de façon connue en soi, en employant une quantité d'amine(s) grasse(s) suffisante pour neutraliser les fonctions acide.

La quantité de sels selon l'invention à ajouter à une huile lubrifiante pour obtenir une efficacité anti-usure est d'au moins 0,01 % par rapport au poids de l'huile et est de préférence comprise entre 0,05 et 0,5 %.

L'huile lubrifiante peut être une huile minérale, un hydrocarbure synthétique, une huile synthétique appartenant aux différentes familles suivantes : glycols, éthers de glycols, esters de glycols, les polyoxyalkylène glycols, leurs éthers et leurs esters, les esters d'acides monocarboxyliques ou polycarboxyliques et de monoalcools ou polyalcools, cette énumération n'étant pas limitative.

Lorsqu'on utilise comme bases lubrifiantes, les coupes pétrolières destinées à la fabrication des huiles moteurs, telles que les bases "Neutral Solvent", on associe avantageusement aux sels selon l'invention des additifs dispersants-détergents traditionnels tels que les alkylaryl sulfonates et les alkylphénates de calcium ou de baryum, ou des dispersants "sans cendres" comme les dérivés succiniques. Les additifs dispersants-détergents favorisent la solubilisation des additifs fluorés dans l'huile sans porter atteinte aux propriétés anti-usure de ces derniers et sans perdre leur propre pouvoir.

L'addition de sels selon l'invention aux huiles formulées contenant déjà des additifs tels que les alkyldithiophosphates de zinc apporte une amélioration sensible du pouvoir anti-usure et une augmentation de la capacité de charge de ces huiles sans perturbation des propriétés apportées par les autres additifs : dispersivité, détergence, pouvoir anti-corrosion, par exemple.

Le remplacement dans les formulations d'huiles pour moteurs à combustion interne de tout ou partie du dithiophosphate de zinc utilisé comme additif anti-usure par 0,1 à 0,2 % de sels selon l'invention permet d'atteindre un niveau de protection contre l'usure égal ou supérieur à celui obtenu avec cet additif traditionnel.

Les sels selon l'invention peuvent donc être utilisés soit en remplacement des alkyldithiophosphates de zinc dans les huiles lubrifiantes pour moteurs à essence ou moteurs diesel, soit en suradditivation dans ces huiles.

L'exemple et les tests suivants illustrent l'invention. Les pourcentages indiqués sont exprimés en poids, sauf mention contraire.

### EXEMPLE

### a) Préparation des mélanges d'acides polyfluorocarboxyliques

Dans un erlen de 1 litre, on disperse 43,7 g de zinc dans une solution de 123,8 g d'acide 10-undécènoïque dans 200 ml de chlorure de méthylène, puis sous agitation on ajoute goutte à goutte 300 g d'iodure de perfluorohexyle. Le mélange réactionnel est chauffé à environ 50°C au début de l'addition jusqu'à l'apparition d'un reflux. Après la fin de l'addition (durée : 2 heures), on maintient sous agitation pendant 15 heures.

Au mélange pâteux ainsi obtenu, on ajoute 300 ml d'une solution aqueuse à 20 % d'acide chlorhydrique pour détruire les sels de zinc, puis on extrait à l'éther la phase organique. La phase éthérée est ensuite lavée deux ou trois fois à l'eau distillée, puis décolorée par traitement à l'aide d'une solution à 10 % de thiosulfate de sodium.

Après élimination de l'éther sous pression réduite, on obtient un produit (A6) qui fond à 50°C environ et qui contient 65 % en moles d'acide 11-(perfluorohexyl)-undécanoïque : C₆F₁₃(CH₂)₁₀COOH et 35 % en moles d'acide 10,11-bis[(perfluorohexyl)méthyl]-eicosanedioïque : 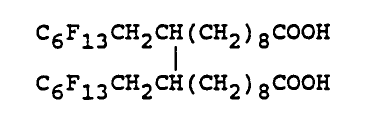
Cette répartition molaire a été déterminée par intégration des signaux obtenus en analyse RMN ¹⁹F.

Par titrage potentiométrique avec de la potasse alcoolique, on a déterminé l'acidité du produit qui est de 2,04 fonctions acide par kilogramme.

En opérant de la même façon à partir de C₄F₉I, de C₈F₁₇I et d'une coupe industrielle de différents iodures CₙF₂ₙ₊₁I ayant la composition suivante :

| **n** | **% molaire** |
|---|---|
| 6 | 52,9 |
| 7 | 0,9 |
| 8 | 31,3 |
| 9 | 0,3 |
| 10 | 8,9 |
| 12 | 2,9 |
| 14 | 0,8 |
| 16 | 0,4 |

et une masse moléculaire moyenne de 578,4, on a obtenu d'autres mélanges d'acides polyfluorocarboxyliques (I) et (II). Le tableau suivant indique les quantités de réactifs utilisés pour ces essais et la répartition molaire des acides mono- et dicarboxyliques dans les produits obtenus.

| **R**_{**F**} **:** | **C**_{**4**}**F**_{**9**} | **C**_{**8**}**F**_{**17**} | **Coupe C**_{**n**}**F**_{**2n+1**} |
|---|---|---|---|
| Quantités utilisées : | | | |
| . R_{F}I (g) | 300 | 300 | 300 |
| . zinc (g) | 56,3 | 35,7 | 33,7 |
| . acide 10-undécènoïque (g) | 159,5 | 101,1 | 95,4 |
| . solvant CH₂Cl₂ (ml) | 200 | 150 | 150 |

| Produit obtenu : | A₄ | A₈ | Aₙ |
|---|---|---|---|
| Monoacide (I) | 72 % | 64 % | 59 % |
| Diacide (II) | 28 % | 36 % | 41 % |
| Zone de fusion (°C) | env.50 | 50-60 | 50-60 |
| Fonctions acide/kg | 2,51 | 1,82 | 1,83 |

### b) Préparation des sels de N,N-bis(2-éthylhexyl)amine

Dans un erlemmeyer, on fait fondre à 60-70°C 120 g du mélange d'acides précédemment obtenu (produit A₆). Après fusion complète et sous agitation, on ajoute 60 g de N,N-bis(2-éthylhexyl)amine, puis on maintient sous agitation pendant 15 minutes pour homogénéiser. On laisse ensuite refroidir à température ambiante.

On obtient ainsi 180 g du sel (ci-après désigné S6) des acides polyfluorocarboxyliques (I) et (II) avec R_{F} = C₆F₁₃.

En opérant d'une façon similaire avec les produits A₄, A₈ et Aₙ du tableau précédent, on obtient les sels correspondants, identifiés ci-après par les abréviations S₄, S₈ et Sₙ respectivement.

### TESTS ANTI-USURE

Le pouvoir anti-usure de compositions lubrifiantes contenant comme huile de base l'huile minérale 200 Neutral Solvent et comme additif un sel selon l'invention a été déterminé à l'aide de la machine 4 billes EP de SHELL dont la description figure dans "Annual Book of ASTM Standards", Part 24 (1979), pages 680-688.

Le test consiste à faire tourner une bille de 12 mm de diamètre avec une vitesse de rotation de 1500 tr/mn sur trois autres billes maintenues immobiles et couvertes de lubrifiant à étudier. Une charge de 40 daN est appliquée par un système de levier qui pousse les trois billes fixes vers la bille supérieure placée dans un mandrin.

L'efficacité anti-usure d'un lubrifiant est déterminée par la valeur moyenne des diamètres des empreintes d'usure sur les trois billes fixes, après une heure de fonctionnement.

Le tableau suivant rassemble les résultats obtenus avec différents sels selon l'invention, utilisés à raison de 0,1 % par rapport au poids d'huile.

| Additif fluoré | Diamètre d'usure en mm | | |
|---|---|---|---|
| | 30°C | 80°C | 120°C |
| Néant | 0,90 | 0,80 | 1,39 |
| S₄ | 0,39 | 0,41 | 0,67 |
| S₆ | 0,39 | 0,40 | 0,39 |
| S₈ | 0,38 | 0,38 | 0,41 |
| Sₙ | 0,42 | 0,42 | 0,42 |

## Revendications

1. Sels d'amine grasse d'un mélange comprenant au moins un acide monocarboxylique de formule :
R_{F}(CH₂)₁₀COOH (I)
et au moins un acide dicarboxylique de formule : 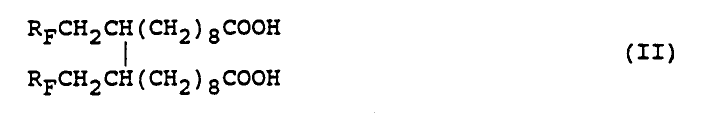 où R_{F} représente un radical perfluoroalkyle, linéaire ou ramifié, contenant de 4 à 16 atomes de carbone.

2. Sels selon la revendication 1, dans lesquels les radicaux R_{F} contiennent de 6 à 12 atomes de carbone.

3. Sels selon la revendication 1 ou 2, dans lesquels la proportion molaire en sel d'acide monocarboxylique (I) est comprise entre 60 et 75 %.

4. Sels selon l'une des revendications 1 à 3, dans lesquels l'amine grasse est choisie parmi les amines aliphatiques ou cycloaliphatiques contenant au total de 12 à 24 atomes de carbone, de préférence de 16 à 20 atomes de carbone, ou un mélange de telles amines.

5. Sels selon l'une des revendications 1 à 4, dans lesquels l'amine grasse est la N,N-bis(2-éthylhexyl)amine.

6. Utilisation des sels selon l'une des revendications 1 à 5 comme additifs anti-usure pour lubrifiants.

7. Lubrifiants, caractérisés en ce qu'ils contiennent un sel selon l'une des revendications 1 à 5.

8. Lubrifiants selon la revendication 7, dans lesquels la teneur en sels est d'au moins 0,01 % en poids et, de préférence, comprise entre 0,05 et 0,5 %.

9. Lubrifiants selon la revendication 7 ou 8, dans lesquels les sels sont associés aux additifs classiques.

## Claims

1. Fatty amine salts of a mixture containing at least one monocarboxylic acid of formula:
R_{F}(CH₂)₁₀COOH (I)
and at least one dicarboxylic acid of formula: 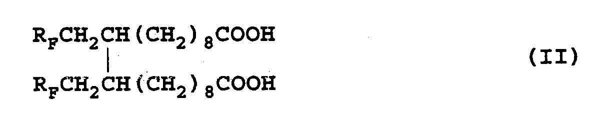 where R_{F} denotes a linear or branched perfluoroalkyl radical containing from 4 to 16 carbon atoms.

2. Salts according to Claim 1, in which the radicals R_{F} contain from 6 to 12 carbon atoms.

3. Salts according to Claim 1 or 2, in which the molar proportion of monocarboxylic acid salt (I) is between 60 and 75 %.

4. Salts according to one of Claims 1 to 3, in which the fatty amine is chosen from aliphatic or cycloaliphatic amines containing in all from 12 to 24 carbon atoms, preferably from 16 to 20 carbon atoms, or a mixture of such amines.

5. Salts according to one of Claims 1 to 4, in which the fatty amine is N,N-bis(2-ethylhexyl)amine.

6. Use of the salts according to one of Claims 1 to 5 as antiwear additives for lubricants.

7. Lubricants characterized in that they contain a salt according to one of Claims 1 to 5.

8. Lubricants according to Claim 7, in which the salt content is at least 0.01 % by weight and preferably between 0.05 and 0.5 %.

9. Lubricants according to Claim 7 or 8, in which the salts are used in combination with conventional additives.

## Patentansprüche

1. Salze von Fettaminen aus einer Mischung, enthaltend mindestens eine Monocarbonsäure der Formel
R_{F}(CH₂)₁₀COOH (I)
und mindestens eine Dicarbonsäure der Formel 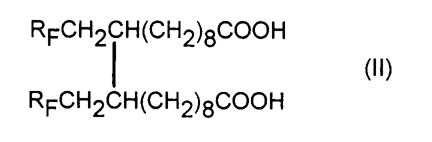 wobei R_{F} einen linearen oder verzweigten Perfluoralkylrest mit 4 bis 16 Kohlenstoffatomen darstellt.

2. Salze nach Anspruch 1, in denen die Reste R_{F} 6 bis 12 Kohlenstoffatome enthalten.

3. Salze nach Anspruch 1 oder 2, in denen die molare Menge an Salz der Monocarbonsäure I zwischen 60 und 75 % beträgt.

4. Salze nach einem der Ansprüche 1 bis 3, in denen das Fettamin ausgewählt ist aus aliphatischen oder cycloaliphatischen Aminen mit insgesamt 12 bis 24 Kohlenstoffatomen, vorzugsweise 16 bis 20 Kohlenstoffatomen, oder einer Mischung solcher Amine.

5. Salze nach einem der Ansprüche 1 bis 4, in denen das Fettamin N,N-Bis-(2-Ethylhexyl)amin ist.

6. Verwendung der Salze nach einem der Ansprüche 1 bis 5 als verschleißfeste Additive für Schmiermittel.

7. Schmiermittel, dadurch gekennzeichnet, daß sie ein Salz nach einem der Ansprüche 1 bis 5 enthalten.

8. Schmiermittel nach Anspruch 7, in denen der Gehalt an den Salzen mindestens 0,01 Gew.-%, vorzugsweise zwischen 0,05 und 0,5 Gew.-%, beträgt.

9. Schmiermittel nach Anspruch 7 oder 8, in denen die Salze mit herkömmlichen Additiven assoziiert sind.
